Europäisches Patentamt

European Patent Office

Office européen des brevets

(11) Publication number: **0 254 378**
**A2**

(12) **EUROPEAN PATENT APPLICATION**

(21) Application number: **87201566.4**

(22) Date of filing: **05.09.83**

(51) Int. Cl.⁴: **C07F 3/02** , **C07F 3/06** ,
**C07C 49/92** , **C07C 45/77**

(30) Priority: **15.09.82 US 418215**

(43) Date of publication of application:
**27.01.88 Bulletin 88/04**

(60) Publication number of the earlier application in
accordance with Art.76 EPC: **0 103 448**

(84) Designated Contracting States:
**AT**

(71) Applicant: **Corning Glass Works**
**Sullivan Park FR-212**
**Corning New York 14831(US)**

(72) Inventor: **Miller, Stephen Bruce**
**67 West Fourth Street**
**Corning New York 14830(US)**
Inventor: **Stewart, Ronald Leroy**
**741 Cardinal Lane**
**Big Flats New York(US)**
Inventor: **Thompson, David Allen**
**159 Stoneybrook Drive**
**Horseheads New York(US)**

(74) Representative: **Kyle, Diana et al**
**ELKINGTON AND FIFE High Holborn House**
**52/54 High Holborn**
**London WC1V 6SH(GB)**

(54) A process for making an organometallic complex.

(57) A process is described for making organometallic complexes suitable for use in the production of glass or ceramic articles by a vapour phase oxidation process.

The complex has the formula M(hfa)₂.nTHF wherein M is Mg or Zn, hfa is hexafluoroacetylacetonate, THF is tetrahydrofuran and n is 2 which is prepared by

(a) providing an adduct of the complex M(hfa)₂ which comprises one or more adducted water molecules;

(b) dissolving the water-containing adduct in excess THF to form a reaction mixture wherein the adduct will react with the THF to replace the adducted molecules by THF;

(c) evaporating the reaction mixture to dryness to form a solid residue; and

(d) collecting the M(hfa)₂.nTHF product from the residue by sublimation of the residue.

# A PROCESS FOR MAKING AN ORGANOMETALLIC COMPLEX

This invention relates to a process for making an organometallic complex and, more particularly, to a process for making a $\beta$-diketonate complex.

A characteristic of the $\beta$-diketonate complexes prepared by the process according to the present invention is that they have a vapour pressure of at least 10 mm (Hg) (13.34 millibars) at a temperature not exceeding 250°C and are suitable for use in the production of glass or ceramic articles by a vapour phase oxidation process which is described and claimed in European Patent Application Published No. 0103448.

Thus, the present invention provides a process for making an organometallic complex of the formula: $M(hfa)_2.nTHF$, wherein M is Mg or Zn, hfa is hexafluoroacetylacetonate, THF is tetrahydrofuran, and n is 2, which comprises the steps of:

(a) providing an adduct of the complex M-$(hfa)_2$ which comprises one or more adducted water molecules;

(b) dissolving the water-containing adduct in excess THF to form a reaction mixture wherein the adduct wil react with the THF to replace the adducted molecules by THF;

(c) evaporating the reaction mixture to dryness to form a solid residue; and

(d) collecting the $M(hfa)_2.nTHF$ product from the residue by sublimation of the residue.

The invention is illustrated by the following Examples.

## Example 1 - $Mg(hfa)_2$

$\beta$-diketonate complexes of the group IIA metal magnesium are prepared by reacting (Hhfa) with a basic magnesium carbonate. A 2.5 g sample of basic magnesium carbonate, $4\ MgCo_3.Mg(OH)_2.nH_2O$ ($n \approx 6$), is suspended in 100 ml of ether with stirring under nitrogen. A 10.41 g sample of (Hhfa) is added to the suspension and the mixture is refluxed for 2 hours. The ether is then separated from a solid residual phase by decantation and evaporated to dryness. Evaporation leaves a residual white powder product identified as the ether-water adduct of magnesium hexafluoroacetylacetonate, $Mg(hfa)_2.1.5Et_2O.H_2O$. This adducted complex melts at about 225°C and may be vaporized by sublimation at 165°C.

To prepare a tetrahydrofuran (THF) adduct of $Mg(hfa)_2$, a 20 ml volume of THF is added to the ether-water adduct produced as described above and the resulting solution is stirred for 18 hours. Rotary evaporation of the solution leaves a white powder residue identified by proton nuclear magnetic resonance (nmr) as $Mg(hfa)_2.4THF$. Sublimation of this compound to a dry ice-cooled cold trap produces the adduct $Mg(hfa)_2.2THF$, a compound having a melting point of approximately 130°C which may be vaporized at 160°C to generate Mg-containing vapours for a vapour phase oxidation process.

## Example 2 - $Zn(hfa)_2.1.5.\ THF$

Following the procedure reported by Chattoraj, et al., J. Inorg. Chem., 28, (1966), pages 1937-1943, the water adduct of $Zn(hfa)_2$ is prepared by reacting Hhfa with zinc oxide. 10 grams of ZnO and 35.2 ml of Hhfa are added to a flask equipped with a condenser, magnetic stirrer and heating mantel, with stirring to disperse the ZnO. 30 ml of $H_2O$ is added, causing the reflux of Hhfa due to the evolution of heat, with stirring being continued until all evidence of reaction has ceased.

An additional 30 ml of water and 200 ml of ether are then added and the mixture is refluxed for 1 hour. After cooling, excess ZnO is removed by filtration, ether layer is separated and dried by adding 4Å ($4 \times 10^-$ ether layer is separated and dried by adding 4Å ($4 \times 10^{-10}$ m) molecular sieves, filtered and the ether is then evaporated to give 55 g of crude $Zn(hfa)_2.2H_2O$ product.

Approximately 10 g of this product is dissolved in THF at room temperature, the solvent is then evaporated and the residue is sublimed at 150°C under vacuum to a dry ice-cooled cold trap. Infrared spectra, nuclear magnetic resonance, thermogravimetric analysis, elemental analysis and differential scanning calorimetry are used to characterize this compound. The compound is presently believed to have the chemical formula $Zn(hfa)_2.2THF$, with a melting temperature of 165°C. It exhibits excellent thermal stability, showing only slight decomposition when maintained at 165°C for about 60 hours.

## Claims

1. A process for making an organometallic complex of the formula: $M(hfa_2).nTHF$, wherein M is Mg or Zn, hfa is hexafluoroacetylacetonate, THF is tetrahydrofuran, and n is 2, which comprises the steps of:

(a) providing an adduct of the complex M-$(hfa)_2$ which comprises one or more adducted water molecules;

(b) dissolving the water-containing adduct in excess THF to form a reaction mixture wherein the adduct will react with the THF to replace the adducted molecules by THF;

(c) evaporating the reaction mixture to dryness to form a solid residue; and

(d) collecting the $M(hfa)_2 \cdot nTHF$ product from the residue by sublimation of the residue.

2. A process according to claim 1, wherein M is Zn and wherein the water-containing adduct is $Zn(hfa)_2 \cdot 2H_2O$.

3. A process according to claim 1, wherein M is Mg and wherein the water containing adduct is $Mg(hfa)_2 \cdot 1.5Et_2O \cdot H_2O$.